Europäisches Patentamt

European Patent Office

Office européen des brevets

(19)

(11) Publication number: **0 119 173**
**A1**

## (12) EUROPEAN PATENT APPLICATION

(21) Application number: **84830059.6**

(22) Date of filing: **08.03.84**

(51) Int. Cl.³: **A 61 F 5/42**

(30) Priority: **11.03.83 IT 936583**

(43) Date of publication of application:
**19.09.84 Bulletin 84/38**

(84) Designated Contracting States:
**AT BE CH DE FR GB LI LU NL SE**

(71) Applicant: **Vignoli, Giancarlo**
**Via Gualandi, 1**
**I-40136 Bologna(IT)**

(71) Applicant: **Coolsaet, Baldovino**
**Via Noordheuvel, 50**
**Wuustwezel(BE)**

(72) Inventor: **Vignoli, Giancarlo**
**Via Gualandi, 1**
**I-40136 Bologna(IT)**

(72) Inventor: **Coolsaet, Baldovino**
**Via Noordheuvel, 50**
**Wuustwezel(BE)**

(74) Representative: **Pederzini, Paolo**
**BUGNION S.p.A. Via Farini n. 37**
**I-40124 Bologna(IT)**

(54) **Rigid-inflatable means for artificially erecting the penis in the case of impotence.**

(57) The rigid-inflatable means for artificially erecting the penis in incurable cases of impotence comprise a tank-pump 6 that allows the filling with liquid of the cavities made in the two parts 3 of two cylinders 1 and 2 implanted in the corpora cavernosa 12. The said liquid passes from the tank-pump 6 into the said parts 3 of the cylinders 1 and 2 causing them to become rigid and thus giving the penis adequate consistence for vaginal penetration.

FIG 3

EP 0 119 173 A1

Rigid-inflatable means for artificially erecting the penis
in the case of impotence

The invention relates to means for artificially erecting
the penis in incurable cases of impotence that comprise
a tank-pump and two cylinders implanted in the corpora
cavernosa.

The erection process of the penis is characterized by
blood passing from the pudendal artery (branch of the hypogastric artery) into the inside of the cavernosa tissue,
the said phenomenon being performed with the mediation of
the autonomous nervous system.

An alteration in the nerves or an obstruction in the vessels entrusted with the movement of the blood brings about
the condition whereby erection is impossible, defined clinically as impotence.

An erection is characterized by the volume of the penis
becoming greater and by an increase in the consistence
thereof, both necessary for adequate vaginal penetration.

Artificial means of the rigid, semi-rigid or pliable, and
of the inflatable type are currently in existence for the
treatment of impotence, the said means always being constituted by two cylindrical elements surgically inserted
inside the corpora cavernosa.

In particular, inflatable means require a tank for liquid
that has to be implanted underneath the abdominal wall
muscles, and mechanism for displacing the liquid from the
tank to the cylinders and vice versa.   This mechanism,
that can be assimilated to a pump, is inserted in the
scrotum and the operation thereof generally requires multiple action and is, therefore, somewhat difficult.

The means forming the subject of the invention can be defined rigid-inflatable means in which both chambers of the cylinders are connected to a pump that also serves as the tank, through the interposition of a valve that can be housed in the scrotum.

In this way, the use is dispensed with of a tank for liquid as is required in the known art and the artificial means are confined purely to the corpora cavernosa and to the scrotum without surgery in the abdominal region being required.

The characteristics of the means forming the subject of the invention will now be outlined more fully in the description that follows of one preferred embodiment that is illustrated on the accompanying drawings and in which :

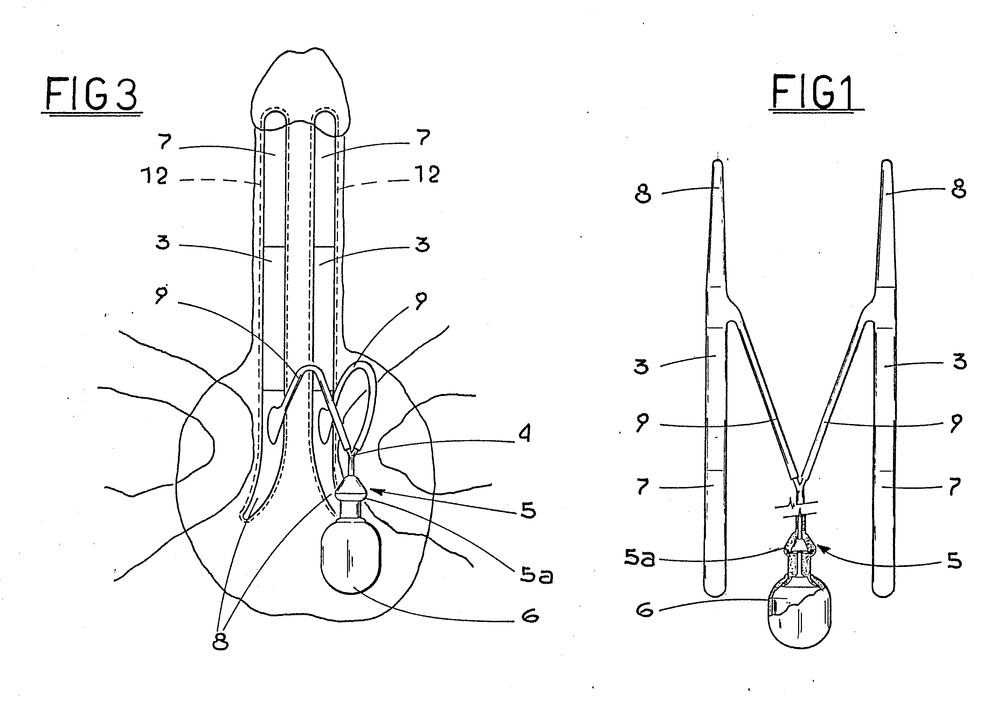

- Figure 1 shows the means in question in a plan view with the various component parts assembled one to the other;
- Figure 2 shows, in a longitudinal section, one of the cylinders of the means seen in a larger scale than in Figure 1;
- Figure 3 shows, diagrammatically, the means in question surgically inserted in the corpora cavernosa and in the scrotum;
- Figure 4 shows, diagrammatically in a longitudinal section in the same scale as in Figure 2, one possible embodiment for the cylinders that is modified compared with that illustrated in Figure 2.

The cylinders 1 and 2 that have to be implanted in the corpora cavernosa, are constituted by a semi-rigid part 7 positioned, during the implantation process, underneath the glans, and by a semi-rigid part 8 from which extends a tube 9 for the filling liquid.   A middle part 3 in between the parts 7 and 8 is constituted by a hollow, flexible, chamber that can be filled with liquid.

The tubes 9 belonging to the cylinders 1 and 2 are joined, through a connector 4, to a valve or valvular system 5 contained in a non-rigid bulb 5a directly connected to a tank-pump 6.

All the components are made out of biocompatible, elastic, material (silastene, polyurethane or similar) that ensures elasticity and flexibility for the chamber 3 (when this is empty) and sufficient rigidity for the rigid parts 7 and 8 that can be constituted by cylinders of the same type of material (though not necessarily of the same composition).

The liquid utilized for the operation of the artificial erection means has to be biocompatible : use is normally made of a physiological solution but silicone oil or similar can also be employed.

The valve 5 normally allows the liquid to pass only in the direction that goes from the tank-pump 6 to the chambers 3; however, the said valve has been designed in such a way that lateral pressure on the bulb 5a inside which the valve is contained, renders it pervious in both directions so that the liquid can return from the chambers 3 to the tank-pump 6.

A description will now be given of the operation of the means forming the subject of the invention.

When non-operative at the time of flaccidity, the liquid that fills what can be defined as the hydraulic system comprising the tank-pump 6, the valve 5, the connector 4, the tubes 9 and the chambers 3, is almost completely contained in the tank 6, while the two chambers 3 are almost empty, leaving maximum positioning freedom for the parts 7 and 8.

When the patient wishes to obtain an erection, the tank pump 6 has to be compressed through one single manual action via the scrotum.   This forces the valve 5 to open and the liquid to flow into the chambers 3, causing them to become rigid and to swell.   The penis is, in this way, made to displace vertically with respect to the abdominal wall similarly to the deviation that occurs with a physiligcal erection.

Thus the penis acquires adequate axial rigidity and consistence to permit normal vaginal penetration.

To bring about detumescence, the patient has to perform one single operation of decompression on the lateral part of the bulb in which the valvular element 5 is contained, and in this way the liquid flows back into the tank-pump 6 rendering the chambers 3 flexible once again and allowing the penis to adopt the optimum flaccidity position.

An example of how the artificial means are implanted is shown in Figure 3.   The method used can be supra-pubic or infrapubic and it involves placing the cylinders 1 and 2 in position inside the corpora cavernosa 12, and the tank-pump 6 with the valve 5 inside the pouch enclosing the testes, obviously with these being retained.

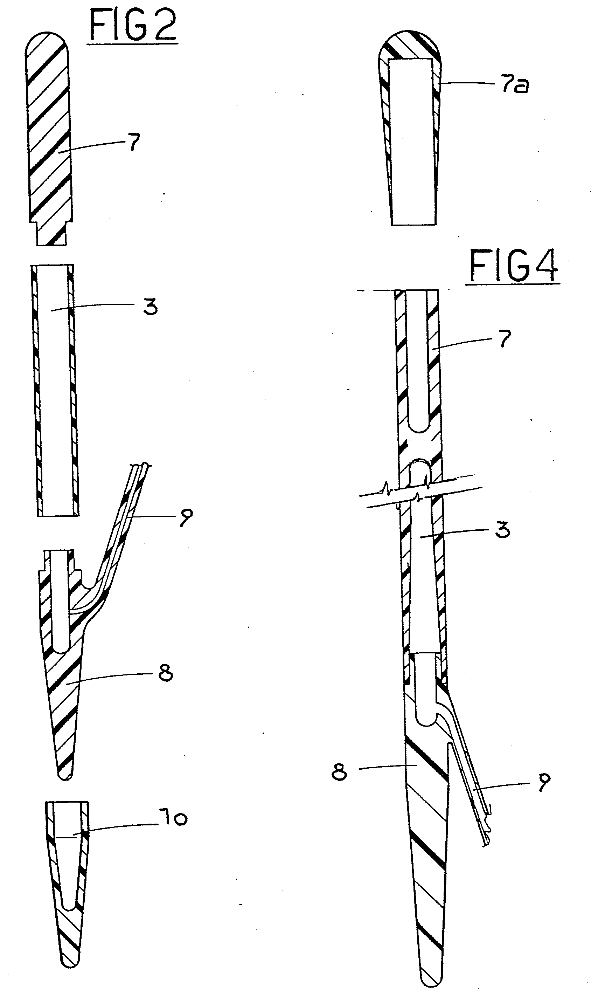

In cases when the length of the cylinders is not sufficient, the use is envisaged of extension pieces (again made of biocompatible elastic material) that are shown at 10 in the example illustrated in Figure 2, and are inserted on to the tapered extremity of the part 8.   The said extension pieces shall be provided in various lengths, to be utilized according to the requirements, keeping to one single basic structure for the cylinders.

The capacity of the tank-pump 6 has been so calculated that when it is compressed, the liquid displaced be sufficient to fill completely the chambers 3 at a pressure that is not dangerous, and at any rate no greater than with 100 - 130 centimetres of $H_2O$.

Advantageously, the part 7 can be made in one piece with the connector 4 and be provided with a sealing cap 7a to be inserted stably on to the said part 7 until it arrives flush with the end border of this.

The part 7 can, in this way, be dimensioned lengthwise at a maximum value and, therefore, be shortened by the surgeon during the implantation operation to suit the said part 7 to the characteristics of the penis of the patient.

According to one possible constructional variant (see Figure 4), the part 3 of the cylinders 1 and 2 is provided with an internal hollow, variable section, chamber, commencing at the engagement area with the part 8. In this way the part 3 can, in the flaccidity condition of the penis, very easily bend back with respect to the part 7 and thereby favour the natural position of the penis in the said condition.

Claims:

1. Rigid-inflatable means for artificially erecting the penis in the case of impotence, of the type comprising cylindrical bodies that can be surgically inserted in the corpora cavernosa, characterized by the fact that each cylindrical body 1 and 2 is composed of at least two semi rigid parts, namely a lower part 8 and an upper part 7, both of which engage with an internally hollow middle part 3 inflatable by means of a liquid in such a way as to adopt consistence in both a radial and in an axial direction, the said liquid being contained in a tank-pump 6 made of material that can be elastically deformed so that it can be squeezed, placed inside the scrotum and connected to the said middle part 3 of each cylindrical body 1 and 2 through a pair of non-rigid linking and branching tubes 9 joined to a duct, also non-rigid, controlled by a valve 5, that can also be housed inside the scrotum; the said valve 5 being contained inside a bulb 5a that can be elastically deformed and is set up to allow the liquid to pass solely in the direction that goes from the tank-pump 6 to the inflatable part 3 of the cylindrical bodies 1 and 2; the said valve 5 being so designed, furthermore, as to be able to be rendered pervious in both directions when the said bulb 5a is made to deform elastically because of pressure being applied manually thereon from the outside as a result of the pouch enclosing the testes being pressed; the said tank-pump 6 being able to contain sufficient a quantity of liquid to give consistence contemporaneously to the middle part 3 of the cylindrical bodies 1 and 2 when the region of the scrotum in which the said tank-pump 6 is contained is pressed manually, even once only.

2. Rigid-inflatable means according to Claim 1, characterized by the fact that the lower part 8 of each cylindrical body 1 and 2 extends axially and is tapered towards the free extremity, and that to the said lower part can be fitted an extension piece 10 provided internally with a seat that mates with at least the end section of the said lower part 8.

3. Rigid-inflatable means according to Claim 1, characterized by the fact that the upper part 7 of each cylindrical body 1 and 2 can be implanted below the glans, is of a tubular structure, and is completed by a capping element 7a inserted tightly on to at least the end part of the said upper part 7, the external lateral surface of which joins, without any break in continuity, with the external surface of the said upper part 7.

4. Rigid-inflatable means according to Claim 1, characterized by the fact that the middle part 3 in between the lower part 8 and the upper part 7 of each cylinder 1 and 2 is made of flexible material in order to permit, in the condition of flaccidity of the penis, the downward flexure thereof.

5. Rigid-inflatable means according to Claim 1 or Claim 4, characterized by the fact that the cavity in the said middle part 3 is of a progressively greater area in the part thereof close to the mating point with the lower part 8 of the corresponding cylinder 1 or 2.

6. Rigid-inflatable means according to Claim 1, characterized by the fact that the said tank-pump 6 contains sufficient a quantity of liquid with which to swell the middle part 3 of the cylindrical bodies 1 and 2 at a pressure not in excess of 100 - 130 centimetres of $H_2O$.

7. Rigid-inflatable means according to Claim 1 or Claim 2, characterized by the fact that the said lower part 8 is made in one piece with the linking and branching tube 9 that enables the middle part 3 of the corresponding cylinder 1 or 2 to be connected to the duct controlled by the said valve 5, the said lower part 8 being provided internally with a counterbore that communicates with the said tube 9 and opens towards the mating area of the said lower part 8 with the middle part 3 of the corresponding cylinder 1 or 2.

8. Rigid-inflatable means according to Claim 1 or Claim 2 or Claim 3, characterized by the fact that all the component parts are made of elastic material.

FIG 3

FIG 1

1/2

0119173

FIG2

FIG4

**0119173**
Application number

EP 84 83 0059

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-3 954 102 (BUUCK)<br>* Whole document * | 1-8 | A 61 F 5/42 |
| | --- | | |
| Y | WO-A-8 000 302 (HAKKY)<br>* Whole document * | 1-8 | |
| | ----- | | |

|  | TECHNICAL FIELDS SEARCHED (Int. Cl. ³) |
|---|---|
| | A 61 F |

The present search report has been drawn up for all claims

| Place of search | Date of completion of the search | Examiner |
|---|---|---|
| THE HAGUE | 08-06-1984 | LOWE D. |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

& : member of the same patent family, corresponding
　　document

EPO Form 1503. 03.82